(19) Europäisches Patentamt
European Patent Office
Office européen des brevets

(11) EP 0 626 366 B1

(12) **FASCICULE DE BREVET EUROPEEN**

(45) Date de publication et mention de la délivrance du brevet:
**14.01.1998 Bulletin 1998/03**

(51) Int Cl.[6]: **C07C 205/22**, C07C 207/04, C07C 205/37, C07C 205/26, C07C 201/10

(21) Numéro de dépôt: **94401124.6**

(22) Date de dépôt: **20.05.1994**

(54) **Procédé de préparation de nitrophénols**

Verfahren zur Herstellung von Nitrophenolen

Process for the preparation of nitrophenols

(84) Etats contractants désignés:
**CH DE ES FR GB IT LI SE**

(30) Priorité: **26.05.1993 FR 9306325**

(43) Date de publication de la demande:
**30.11.1994 Bulletin 1994/48**

(73) Titulaire: **RHONE-POULENC CHIMIE**
**92408 Courbevoie Cédex (FR)**

(72) Inventeurs:
- **Metivier, Pascal**
  **F-69110 Ste Foy les Lyon (FR)**
- **Bernard, Laurent**
  **F-69200 Venissieux (FR)**

(74) Mandataire: **Dutruc-Rosset, Marie-Claude et al**
**RHONE-POULENC CHIMIE,**
**Direction de la Propriété Industrielle,**
**25, Quai Paul Doumer**
**92408 Courbevoie Cédex (FR)**

(56) Documents cités:
**DE-A- 1 493 827** **DE-A- 1 965 384**
**GB-A- 1 165 637** **US-A- 1 502 849**

- **CHEMICAL ABSTRACTS, vol. 089, no. 3, 17 Juillet 1978, Columbus, Ohio, US; abstract no. 023929, M. VITAN et al, "Continuous preparation of p-nitrophenol"; & RO-A-60 143**
- **CHEMICAL ABSTRACTS, vol. 088, no. 25, 19 Juin 1978, Columbus, Ohio, US; abstract no. 190368, S. Z. OROSZ et al, "p-Nitrosophenol in a continuous process"; & RO-A-60 020**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 626 366 B1

Printed by Jouve, 75001 PARIS (FR)

## Description

La présente invention a pour objet un procédé de préparation de nitrophénols. Plus particulièrement, l'invention concerne la préparation du p-nitrophénol.

Il existe de très nombreuses voies d'accès au p-nitrophénol.

En particulier, on a décrit dans US-A 3 517 075, un procédé de préparation du p-nitrophénol par oxydation du p-nitrosophénol. Dans une première étape, on prépare tout d'abord le p-nitrosophénol par nitrosation du phénol à l'aide de l'acide nitreux, en présence d'acide sulfurique, puis, dans une deuxième étape, on oxyde le p-nitrosophénol en p-nitrophénol. Un inconvénient majeur d'un tel procédé est qu'il y a précipitation du p-nitrosophénol. Or ce dernier est un composé instable qui présente des risques d'explosion thermique importants.

La présente invention se propose de réaliser la préparation du p-nitrophénol selon un procédé qui évite ce problème de sécurité.

Plus précisément, la présente invention a pour objet un procédé de préparation d'un p-nitrophénol par nitrosation d'un composé phénolique en présence d'acide sulfurique, puis oxydation du p-nitrosophénol par l'acide nitrique, caractérisé par le fait que dans la première étape de nitrosation, la concentration en acide sulfurique est au moins égale à 60 % et dans la deuxième étape, en fin de réaction d'oxydation, la concentration en acide sulfurique est au plus égale à 80 % ce qui permet de précipiter le p-nitrophénol qui est ensuite séparé.

Le procédé de l'invention, grâce aux contrôles de la concentration en acide sulfurique dans les deux étapes de nitrosation et d'oxydation permet:

- d'une part de pallier aux problèmes de l'explosivité du p-nitrosophénol car en choisissant une concentration de l'acide sulfurique supérieure à 60 % dans la première étape, le p-nitrosophénol est alors soluble,
- et d'autre part de récupérer le p-nitrophénol précipité en fin d'étape d'oxydation, lorsque la concentration de l'acide sulfurique est inférieure à 80 % ; celui-ci devenant soluble à des concentrations plus élevées.

La demanderesse a trouvé, de manière tout à fait surprenante, que le p-nitrosophénol était soluble dans les conditions définies par l'invention.

L'invention s'applique tout particulièrement au phénol mais aussi à tout composé aromatique, porteur d'un groupe OH dont la position en para du groupe OH est libre.

Parmi les composés aromatiques hydroxylés, l'invention s'applique tout particulièrement à ceux qui répondent à la formule générale (I) :

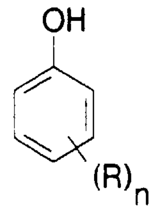
(I)

dans ladite formule (I) :

- R représente un atome d'hydrogène, un radical alkyle ou alkoxy ayant de 1 à 4 atomes de carbone, de préférence méthyle ou éthyle ; un radical alkyle perfluoré ayant de 1 à 4 atomes de carbone ; un atome d'halogène, de préférence le chlore, le brome ou le fluor,
- n est un nombre égal à 0,1 ou 2.

La présente invention n'exclut pas la présence d'autres substituants sur le cycle aromatique dans la mesure où ils n'interfèrent pas avec les réactions du procédé de l'invention. En particulier, il est possible qu'il y ait présence de groupes fonctionnels ou d'atomes d'halogène sur les chaînes alkyle portées par le noyau benzénique ou que ces chaînes alkyle soient interrompues par un hétéroatome tel que l'oxygène, l'azote ou le soufre.

Dans l'exposé qui suit de la présente invention, on utilisera le terme de "phénol" de manière générique. Il désignera aussi bien le phénol que tous les composés aromatiques hydroxylés dénommés composés phénoliques, en particulier ceux répondant à la formule (I).

Conformément au procédé de l'invention, on effectue dans une première étape, la nitrosation du phénol, en présence d'acide sulfurique.

Comme mentionné précédemment, la quantité d'acide sulfurique mise en oeuvre est particulièrement critique.

La concentration en acide sulfurique dans le milieu réactionnel, exprimée par le rapport pondéral acide sulfurique/acide sulfurique + eau, est au moins égale à 60 % en poids.

Avantageusement, la concentration en acide sulfurique est comprise entre 60 % et 90 %. Elle est choisie de préférence, entre 70 % et 80 %.

La présence d'eau dans l'étape de nitrosation n'est pas gênante dans la mesure où sa teneur est telle que la concentration de l'acide sulfurique soit respectée.

L'agent de nitrosation est toute source de $NO^+$. Ainsi, on peut partir du dioxyde d'azote $NO_2$, de l'anhydride azoteux $N_2O_3$, du peroxyde d'azote $N_2O_4$, de l'oxyde d'azote NO associé à un agent oxydant tel que, par exemple, l'acide nitrique, le dioxyde d'azote ou l'oxygène. Dans le cas où l'agent de nitrosation est gazeux dans les conditions réactionnelles, on le fait buller dans le milieu.

On peut également faire appel à l'acide nitreux, à un sulfate de nitrosyle ou nitrose ou à un sel nitreux, de préférence un sel de métal alcalin, et encore plus préférentiellement, le sodium.

La quantité d'agent de nitrosation mise en oeuvre peut varier largement. Lorsqu'elle est exprimée par le rapport molaire phénol/agent de nitrosation défini en $NO^+$, elle est au moins égale à la quantité stoechiométrique mais il est préférable qu'elle soit mise en oeuvre en un excès pouvant atteindre 500 % de la quantité stoechiométrique, et de préférence, compris entre 150 % et 300 %.

En ce qui concerne la concentration du substrat phénolique dans le milieu réactionnel, elle est de préférence comprise entre 2 % et 20 % en poids.

Le phénol est introduit généralement sous forme liquide. Il peut donc, être soit introduit à l'état fondu, soit en mélange avec de l'eau. Dans ce dernier cas, des mélanges comportant de 60 % à 90 % de phénol conviennent bien. Il y a lieu de veiller que la teneur en eau dans le milieu réactionnel doit être telle que la concentration de l'acide sulfurique soit respectée.

Dans l'étape suivante, on effectue l'oxydation du p-nitrosophénol par l'acide nitrique.

Il est également possible de faire appel à un précurseur de l'acide nitrique tel que par exemple le peroxyde d'azote.

On fait appel à une solution aqueuse d'acide nitrique ayant une concentration indifférente pouvant varier entre 30 % et 100 %. Toutefois, une concentration comprise entre 60 % et 100 % est préférée.

La quantité d'acide nitrique exprimée par le rapport molaire phénol/acide nitrique varie généralement entre 0,9 et 1,2, de préférence, entre 0,95 et 1,05.

Comme indiqué précédemment, la quantité d'acide sulfurique mise en oeuvre doit être particulièrement contrôlée dans cette étape.

La concentration en acide sulfurique est inférieure ou égale à 80 %. La limite inférieure ne présente aucun caractère critique. Elle est avantageusement comprise entre 50 % et 80 %, et de préférence, entre 65 % et 75 %.

Au cours de la réaction de nitrosation, il peut y avoir formation d'eau. Lors de la réaction d'oxydation, il peut être nécessaire d'en ajouter afin d'atteindre les concentrations d'acide sulfurique précitées. Le plus souvent, l'eau est ajoutée en même temps que l'acide nitrique.

Le procédé de l'invention est avantageusement conduit à une température comprise entre 0 et 40°C, de préférence entre 10°C et 30°C.

Le procédé de l'invention est généralement mis en oeuvre sous pression atmosphérique mais peut l'être également sous pression légérement réduite comprise, par exemple, entre 500 et 760 mm de mercure [66500 et 101080 Pa] ou dans des conditions de surpression pouvant atteindre, par exemple, 5 bars.

Selon une variante préférée du procédé de l'invention, on conduit l'étape de nitrosation sous atmosphère contrôlée de gaz inertes. On peut établir une atmosphère de gaz rares, de préférence l'argon mais il est plus économique de faire appel à l'azote.

D'un point de vue pratique, le procédé de l'invention est facile à mettre en oeuvre car il ne nécessite pas d'avoir recours à un appareillage spécifique.

Pratiquement, le procédé de l'invention peut être mis en oeuvre de la manière décrite ci-après.

On charge les différents constituants du mélange réactionnel dans l'appareillage choisi.

Plusieurs modes de mises en oeuvre peuvent être envisagés.

Une première variante consiste à charger d'abord la solution d'acide sulfurique puis d'ajouter ensuite en parallèle le phénol et l'agent de nitrosation.

Selon une autre variante, on introduit la solution d'acide sulfurique et l'agent de nitrosation puis l'on ajoute le phénol, de préférence par portions ou de manière continue par coulée.

Une autre variante réside dans le fait d'introduire en parallèle, sur un pied de cuve, le phénol d'un côté et l'acide sulfurique et l'agent de nitrosation de l'autre.

Après mise en oeuvre des réactifs, on maintient le mélange réactionnel dans la zone de température précitée. Il peut être opportun de refroidir le mélange réactionnel.

Dans l'étape suivante d'oxydation, on introduit ensuite l'acide nitrique dans le milieu réactionnel comprenant le p-nitrosophénol. On peut l'ajouter en une seule fois ou progressivement par portions ou de manière continue, par coulée. Il est également possible d'introduire l'acide nitrique dès le départ, par exemple en parallèle avec l'addition du phénol.

On maintient la température du mélange réactionnel, dans la zone de température précitée.

Selon un autre mode d'exécution de l'invention, il est possible de former l'acide nitrique in situ à partir du peroxyde d'azote qui sert à la fois d'agent de nitrosation dans la première étape et de précurseur d'acide nitrique dans la deuxième étape. A cet effet, le peroxyde d'azote est introduit dès le départ et dans la deuxième étape, est chauffé à une température entre 20°C et 40°C.

Compte-tenu de la concentration du mélange réactionnel en acide sulfurique, le p-nitrophénol précipite.

Le précipité obtenu est séparé selon les techniques classiques de séparation solide/liquide, de préférence par filtration.

On lave le précipité obtenu, de préférence, par une solution d'acide sulfurique ayant la même concentration que celle définie à l'étape d'oxydation.

Pour débarrasser le précipité de ses impuretés, on peut effectuer un autre lavage à l'eau.

Les eaux-mères recueillies après chaque séparation peuvent être recyclées. Elles sont riches en agent de nitrosation car celui-ci est régénéré au cours de l'oxydation.

Le procédé de l'invention permet d'obtenir essentiellement du p-nitrophénol car la quantité d'o-nitrophénol est généralement inférieure à 5 %.

De plus, selon le procédé de l'invention, le p-nitrophénol précipite préférentiellement par rapport à l'o-nitrophénol et aux dinitrophénols obtenus en tant qu'impuretés.

Le procédé de l'invention permet d'obtenir un composé p-nitrophénolique et plus particulièrement le p-nitrophénol qui peut servir comme produit intermédiaire pour la préparation du N-acétyl p-aminophénol qui est obtenu par réduction du p-nitrophénol puis acétylation du p-aminophénol obtenu.

Ces opérations sont connues et l'on peut se référer, par exemple, à l'ouvrage KIRK-OTHMER [Encyclopedia of Chemical Technology 2, 3ème édition]

La réduction du p-nitrophénol est effectuée d'une manière classique par mise en contact avec l'hydrogène, en présence d'un catalyseur d'hydrogénation, de préférence, un métal noble platine ou palladium. Ledit métal peut être déposé sur support : charbon, noir d'acétylène, silice, alumine etc...

L'acétylation effectuée de manière connue, est réalisée de préférence, avec l'anhydride acétique.

Les exemples qui suivent, illustrent l'invention sans toutefois en limiter la portée.

$$TT = \frac{\text{nombre de moles de phénol transformées}}{\text{nombre de moles de phénol introduites}} \%$$

$$RT = \frac{\text{nombre de moles de nitrophénol formées}}{\text{nombre de moles de phénol transformées}} \%$$

EXEMPLES

Exemple 1:

Dans un réacteur de 1 litre muni d'une double enveloppe, d'une agitation mécanique et d'une sonde de température, on charge 500 ml d'une solution aqueuse d'acide sulfurique à 80 % en poids, et l'on ajoute 97,4 g d'une solution aqueuse de $NaNO_2$ à 42,5 % (soit 0,6 mol de $NaNO_2$).

On refroidit le mélange réactionnel à 10°C et l'on coule alors en 40 mn et en maintenant la température à 10°C, 33,7 g d'une solution aqueuse de phénol à 80 % (soit 0,286 mol de phénol).

On maintient ensuite le mélange réactionnel 5 mn à 10°C, puis l'on coule en 10 mn, 30,1 g d'une solution aqueuse d'acide nitrique à 67 % en poids (0,32 mol), tout en maintenant la température à 10°C.

Le mélange réactionnel devient hétérogène.

On maintient le mélange réactionnel à 10°C, puis l'on filtre.

Le gâteau est lavé par 2 fois, 50 g d'une solution aqueuse d'acide sulfurique à 70 %, puis par de l'eau et séché sous vide de la trompe à eau.

On récupère ainsi 32,94 g d'un produit gris clair titrant 95,2 % en p-nitrophénol. Le filtrat ainsi que les eaux de lavage sont récupérés et dosés par chromatographie liquide haute performance.

Les résultats obtenus sur la réaction sont les suivants :

- $TT_{phénol}$ = 100 %
- $RT_{p\text{-}nitrophénol}$ = 92 %
- $RT_{o\text{-}nitrophénol}$ = 0,6 %
- $RT_{dinitrophénols}$ = 0,93 %

Exemple 2 :

Dans un réacteur de 250 ml muni d'une agitation mécanique et d'un système de régulation de température, on charge 100 ml (173 g) d'une solution aqueuse d'acide sulfurique à 80 %.

On ajoute 13 ml (15,3 g) d'une solution aqueuse de nitrite de sodium à 27 % (0,06 mol de $NaNO_2$).

On amène le milieu réactionnel à 10°C.

On coule ensuite en 40 mn, 6,7 ml d'une solution aqueuse de phénol à 80 % (0,0605 mol de phénol) tout en régulant la température à 10°C.

Après 2 minutes de coulée du phénol, on coule alors simultanément, et pendant 38 mn, 6,9 ml d'une solution aqueuse d'acide nitrique à 40 % (0,550 mol d'acide nitrique).

Le milieu réactionnel devient hétérogène.

On maintient le milieu réactionnel 5 mn à 10°C et l'on filtre à froid.

Le gâteau est lavé par deux fois, 30 ml d'une solution aqueuse d'acide sulfurique à 70 %, puis trois fois, par de l'eau (30 ml) puis séché sous vide.

On obtient alors 5,54 g d'un produit solide titrant 95 % en p-nitrophénol.

Les filtrats et eaux de lavages sont réunis et dosés.

Les résultats obtenus sur la réaction sont les suivants:

- $TT_{phénol}$ = 100 %
- $RT_{p\text{-}nitrophénol}$ = 83,6 %
- $RT_{o\text{-}nitrophénol}$ = 6,7 %
- $RT_{dinitrophénols}$ = 0,7 %
- $RT_{p\text{-}nitrosophénol}$ = 6,8 %

Exemple 3 :

Les conditions de réaction sont exactement identiques aux conditions de l'exemple 2, à l'exception de la température qui est maintenue à 26°C tout au long de l'essai.

Les résultats obtenus sont les suivants:

- $TT_{phénol}$ = 100 %
- $RT_{p\text{-}nitrophénol}$ = 62,5 %
- $RT_{o\text{-}nitrophénol}$ = 5,4 %
- $RT_{dinitrophénols}$ = 1,3 %
- $RT_{p\text{-}nitrosophénol}$ = 12,0 %

Exemple 4 :

Dans un réacteur de 250 ml muni d'une double enveloppe et d'une agitation mécanique, on charge en pied 100 ml d'une solution aqueuse d'acide sulfurique à 80 % et l'on ajoute 19,5 g d'une solution aqueuse de nitrite de sodium à 42,5 % (0,12 mol de $NaNO_2$).

On coule ensuite en 40 mn, 7,07 g d'une solution aqueuse de phénol à 80 % (0,0602 mol) tout en maintenant la température à 25°C.

On maintient alors le mélange réactionnel à 25°C pendant 5 mn puis l'on coule en 15 mn, 5,44 g d'une solution aqueuse d'acide nitrique à 67 % (0,058 mol).

On maintient le mélange pendant 5 mn, à 25°C.

Le milieu est hétérogène solide/liquide.

Le dosage par CLHP des différentes espèces donne les résultats suivants :

- $TT_{phénol}$ = 100 %
- $RT_{p\text{-}nitrophénol}$ = 89,5 %
- $RT_{o\text{-}nitrophénol}$ = 0,75 %
- $RT_{dinitrophénols}$ = 2,05 %

Exemple 5 :

Les conditions sont identiques à celles de l'exemple 4 à l'exception de la température qui est de 10°C.

La charge d'acide nitrique est effectuée en 2 fois.

0,7 g d'acide nitrique (0,0074 mol) sont ajoutés au pied d'acide sulfurique et de nitrite de sodium avant coulée du phénol, et 5, 6 g d'acide nitrique (0,0595 mol) sont coulés après la coulée de phénol.

Le milieu réactionnel final est hétérogène.

Le milieu est filtré après dosage des phases.

Les résultats obtenus sont les suivants :

- $TT_{phénol}$ = 100 %
- $RT_{p\text{-}nitrophénol}$ = 91,1 %
- $RT_{o\text{-}nitrophénol}$ = 1,5%
- $RT_{dinitrophénols}$ = 0,8 %

Exemple 6 :

Dans un réacteur de 1 litre muni d'une double enveloppe et d'une agitation mécanique, on introduit 904 g d'une solution aqueuse d'acide sulfurique à 70 %, et 18,5 g d'une solution d'acide nitrique à 68 % (0,2 mol $HNO_3$).

On introduit dans le mélange liquide sous agitation, 13 g d'oxyde d'azote NO gazeux (0,43 mol).

On amène ce mélange réactionnel à 10°C, puis on ajoute 35,2 g d'une solution aqueuse de phénol 80 % en 40 mn, tout en maintenant la température à 10°C, puis on maintient le milieu réactionnel, 5 mn à 10°C et l'on coule ensuite 28,2 g (0,3 mol) d'une solution aqueuse d'acide nitrique à 68 % en 15 mn dans le milieu réactionnel et l'on maintient 5 mn à 10°C.

On filtre alors le milieu réactionnel.

On obtient 15,8 g d'un solide rouge sombre. Ce solide ainsi que les jus-mères restants, sont dosés par chromatographie liquide haute performance.

Les résultats obtenus sur la réaction sont les suivants :

- $TT_{phénol}$ = 100 %
- $RT_{p\text{-}nitrophénol}$ = 61 %
- $RT_{o\text{-}nitrophénol}$ = 4,6 %
- $RT_{dinitrophénols}$ = 8,8 %

Exemple 7 :

Dans un réacteur double enveloppe muni d'une agitation mécanique, on charge en pied, 440,5 g d'une solution aqueuse d'acide sulfurique à 69 %, 40,5 g de sulfate acide de nitrosyle $NO,OSO_3H$ (0,3 mol).

On refroidit le mélange réactionnel à 10°C et l'on coule 17,7 g d'une solution aqueuse de phénol à 80 % (0,15 mol) sur le mélange réactionnel en 40 mn, tout en maintenant la température à 10°C.

On maintient alors le mélange réactionnel pendant 5 mn à 10°C et l'on coule alors 13,9 g d'une solution aqueuse d'acide nitrique à 68 % (0,15 mol) sur le mélange réactionnel en 15 mn, tout en maintenant la température à 10°C.

On filtre alors le mélange réactionnel, on lave le gâteau par 2 fois 30 ml d'une solution aqueuse d'acide sulfurique à 70 %, puis par 3 fois 30 ml d'eau, et l'on sèche le produit sous vide. On obtient 18 g de précipité jaune clair, constitué essentiellement de p-nitrophénol.

Le dosage des différentes phases donne le bilan suivant:

- $TT_{phénol}$ = 100 %
- $RT_{p\text{-}nitrophénol}$ = 88 %
- $RT_{o\text{-}nitrophénol}$ = 1,1 %
- $RT_{dinitrophénols}$ = 1,75 %

Exemple 8 :

Dans un réacteur double enveloppe muni d'une agitation mécanique, on charge en pied, 440 g d'une solution aqueuse d'acide sulfurique à 70 % et 40,5 g de sulfate acide de nitrosyle (0,3 mol). On coule 17,7 g d'une solution aqueuse de phénol à 80 % (0,15 mol) en 10 minutes, tout en maintenant la température à 25°C, puis on maintient le mélange réactionnel 5 mn à 25°C et l'on ajoute ensuite 14,5 g d'une solution aqueuse d'acide nitrique à 68 % (0,156 mol) en 15 mn tout en maintenant la température à 25°C.

On maintient 5 mn à 25°C puis on filtre le mélange réactionnel.

On lave le gâteau par 2 fois 30 ml d'une solution aqueuse d'acide sulfurique à 70 %, puis par 3 fois 30 ml d'eau.

On obtient 12,1 g d'un solide gris titrant 98 % en p-nitrophénol.

Les différentes phases sont dosées par chromatographie liquide haute performance et le bilan est le suivant :

- $TT_{phénol}$ = 100 %
- $RT_{p\text{-}nitrophénol}$ = 90 %
- $RT_{o\text{-}nitrophénol}$ = 0,2 %
- $RT_{dinitrophénols}$ = 3,38 %

**Revendications**

1. Procédé de préparation d'un p-nitrophénol par nitrosation d'un composé phénolique en présence d'acide sulfurique, puis oxydation du p-nitrosophénol par l'acide nitrique, caractérisé par le fait que dans la première étape de nitrosation, la concentration en acide sulfurique est au moins égale à 60 % et dans la deuxième étape, en fin de réaction d'oxydation, la concentration en acide sulfurique est au plus égale à 80 %, ce qui permet de précipiter le p-nitrophénol qui est ensuite séparé.

2. Procédé selon la revendication 1 caractérisé par le fait que le composé phénolique répond à la formule générale (I) :

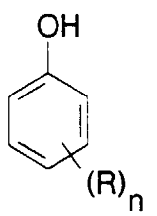
(I)

dans ladite formule (I) :

- R représente un atome d'hydrogène, un radical alkyle ou alkoxy ayant de 1 à 4 atomes de carbone, de préférence méthyle ou éthyle ; un radical alkyle perfluoré ayant de 1 à 4 atomes de carbone ; un atome d'halogène, de préférence le chlore, le brome ou le fluor,
- n est un nombre égal à 0,1 ou 2.

3. Procédé selon l'une des revendications 1 et 2 caractérisé par le fait que le composé phénolique est le phénol.

4. Procédé selon l'une des revendications 1 à 3 caractérisé par le fait que la concentration en acide sulfurique dans l'étape de nitrosation est comprise entre 60 % et 90 %, de préférence, entre 70 % et 80 %.

5. Procédé selon l'une des revendications 1 à 4 caractérisé par le fait que l'agent de nitrosation est toute source de $NO^+$, de préférence l'oxyde d'azote NO associé à un agent oxydant, le dioxyde d'azote $NO_2$, l'anhydride azoteux $N_2O_3$, le peroxyde d'azote $N_2O_4$, l'acide nitreux, le sulfate de nitrosyle ou un sel nitreux, de préférence un sel de métal alcalin, de préférence, le sodium.

6. Procédé selon l'une des revendications 1 à 5 caractérisé par le fait que la quantité d'agent de nitrosation mise en oeuvre est au moins égale à la quantité stoechiométrique mais de préférence, en un excès pouvant atteindre 500 % de la quantité stoechiométrique, et de préférence, compris entre 150 % et 300 %.

7. Procédé selon l'une des revendications 1 à 6 caractérisé par le fait que la concentration du substrat phénolique dans le milieu réactionnel, est comprise entre 2 % et 20 % en poids.

8. Procédé selon l'une des revendications 1 à 7 caractérisé par le fait que la concentration en acide sulfurique dans l'étape d'oxydation est comprise entre 50 % et 80 %, de préférence, entre 65 % et 75 %.

9. Procédé selon l'une des revendications 1 à 8 caractérisé par le fait que l'on met en oeuvre une solution aqueuse d'acide nitrique ou d'un précurseur de l'acide nitrique, de préférence, le peroxyde d'azote.

10. Procédé selon l'une des revendications 1 à 9 caractérisé par le fait que la concentration de la solution aqueuse d'acide nitrique est comprise entre 30 % et 100 %, de préférence entre 60 % et 100% en poids.

11. Procédé selon l'une des revendications 1 à 10 caractérisé par le fait que la quantité d'acide nitrique exprimée par le rapport molaire phénol/acide nitrique varie entre 0,9 et 1,2, de préférence, entre 0,95 et 1,05.

12. Procédé selon l'une des revendications 1 à 11 caractérisé par le fait que la température est comprise entre 0 et 40°C, de préférence entre 10°C et 30°C.

13. Procédé selon l'une des revendications 1 à 12 caractérisé par le fait que dans l'étape de nitrosation du phénol, on charge d'abord la solution d'acide sulfurique puis l'on ajoute ensuite en parallèle le phénol et l'agent de nitrosation ; ou bien l'on introduit la solution d'acide sulfurique et l'agent de nitrosation puis l'on ajoute le phénol ; ou bien l'on introduit en parallèle, sur un pied de cuve, le phénol d'un côté et l'acide sulfurique et l'agent de nitrosation de l'autre.

14. Procédé selon l'une des revendications 1 à 13 caractérisé par le fait que dans l'étape suivante d'oxydation, on introduit l'acide nitrique en une seule fois ou progressivement par portions ou de manière continue, par coulée, dans le milieu réactionnel comprenant le p-nitrosophénol.

**15.** Procédé selon l'une des revendications 1 à 14 caractérisé par le fait que l'on ajoute l'acide nitrique dès le départ, de préférence en parallèle avec l'addition du phénol.

**16.** Procédé selon l'une des revendications 1 à 15 caractérisé par le fait que l'on forme l'acide nitrique in situ à partir du peroxyde d'azote qui est chauffé à une température entre 20°C et 40°C.

**17.** Procédé de préparation du N-acétyl p-aminophénol caractérisé par le fait que, dans une première étape, on effectue la préparation du p-nitrophénol selon le procédé décrit dans l'une des revendications 1 à 16 et, dans une étape suivante, on effectue la réduction du p-nitrophénol puis l'acétylation du p-aminophénol obtenu.

**Patentansprüche**

**1.** Verfahren zur Herstellung eines p-Nitrophenols mittels Nitrosierung einer Phenolverbindung in Gegenwart von Schwefelsäure, anschließender Oxidation des p-Nitrosophenols mit Salpetersäure, dadurch gekennzeichnet, daß in dem ersten Verfahrensschritt der Nitrosierung die Konzentration an Schwefelsäure mindestens 60 % beträgt und in dem zweiten Verfahrensschritt, am Ende der Oxidationsreaktion, die Konzentration an Schwefelsäure höchstens 80 % beträgt, was die Ausfällung des p-Nitrophenols ermöglicht, das anschließend abgetrennt wird.

**2.** Verfahren nach Anspruch 1, dadurch gekennzeichnet, daß die Phenolverbindung der allgemeinen Formel (I)

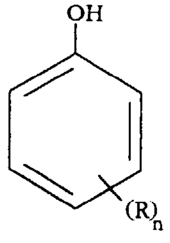

(I)

entspricht, wobei in der Formel (I):

- R ein Wasserstoffatom, einen Alkyl- oder Alkoxyrest mit 1 bis 4 Kohlenstoffatomen, vorzugsweise Methyl oder Ethyl; einen perfluorierten Alkylrest mit 1 bis 4 Kohlenstoffatomen; ein Halogenatom, vorzugsweise Chlor, Brom oder Fluor, darstellt;
- n eine Zahl von 0, 1 oder 2 ist.

**3.** Verfahren nach Anspruch 1 oder 2, dadurch gekennzeichnet, daß die Phenolverbindung Phenol ist.

**4.** Verfahren nach einem der Ansprüche 1 bis 3, dadurch gekennzeichnet, daß die Konzentration an Schwefelsäure in dem Nitrosierungsschritt 60 % bis 90 %, vorzugsweise 70 % bis 80 %, beträgt.

**5.** Verfahren nach einem der Ansprüche 1 bis 4, dadurch gekennzeichnet, daß das Nitrosierungsreagenz jede beliebige $NO^+$-Quelle ist, vorzugsweise Stickstoffmonoxid NO in Verbindung mit einem Oxidationsmittel, Stickstoffdioxid $NO_2$, Distickstofftrioxid $N_2O_3$, Distickstofftetroxid (Stickstoffperoxid) $N_2O_4$, salpetrige Säure, Nitrosylsulfat oder ein Salz des zweiwertigen Stickstoffs, vorzugsweise ein Alkalimetallsalz, insbesondere ein Natriumsalz.

**6.** Verfahren nach einem der Ansprüche 1 bis 5, dadurch gekennzeichnet, daß die verwendete Menge an Nitrosierungsreagenz mindestens der stöchiometrischen Menge entspricht, jedoch vorzugsweise in einem Überschuß vorliegt, der 500 % der stöchiometrischen Menge erreichen kann, vorzugsweise 150 % bis 300 %.

**7.** Verfahren nach einem der Ansprüche 1 bis 6, dadurch gekennzeichnet, daß die Konzentration des Phenolsubstrats in dem Reaktionsmilieu zwischen 2 Gew.-% und 20 Gew.-% liegt.

**8.** Verfahren nach einem der Ansprüche 1 bis 7, dadurch gekennzeichnet, daß die Konzentration an Schwefelsäure in dem Oxidationsverfahrensschritt zwischen 50 % und 80 %, vorzugsweise zwischen 65 % und 75 %, liegt.

**9.** Verfahren nach einem der Ansprüche 1 bis 8, dadurch gekennzeichnet, daß man eine wäßrige Lösung von Salpetersäure oder eines Vorläufers der Salpetersäure verwendet, vorzugsweise Stickstoffperoxid (Distickstofftetroxid).

**10.** Verfahren nach einem der Ansprüche 1 bis 9, dadurch gekennzeichnet, daß die Konzentration der wäßrigen Salpetersäurelösung zwischen 30 Gew.-% und 100 Gew.-%, vorzugsweise zwischen 60 Gew.-% und 100 Gew.-%, liegt.

**11.** Verfahren nach einem der Ansprüche 1 bis 10, dadurch gekennzeichnet, daß die Menge an Salpetersäure, berechnet als Molverhältnis Phenol/Salpetersäure, zwischen 0,9 und 1,2 variiert, vorzugsweise zwischen 0,95 und 1,05.

**12.** Verfahren nach einem der Ansprüche 1 bis 11, dadurch gekennzeichnet, daß die Temperatur zwischen 0 °C und 40 °C, vorzugsweise zwischen 10 °C und 30 °C, liegt.

**13.** Verfahren nach einem der Ansprüche 1 bis 12, dadurch gekennzeichnet, daß man bei dem Verfahrensschritt der Nitrosierung des Phenols zunächst die Schwefelsäure vorlegt, anschließend gleichzeitig das Phenol und das Nitrosierungsreagenz hinzugibt; oder auch daß man die Schwefelsäurelösung und das Nitrosierungsreagenz hinzugibt, anschließend das Phenol zusetzt; oder auch daß man gleichzeitig am Fuß des Behältnisses das Phenol einerseits und die Schwefelsäure und das Nitrosierungsreagenz andererseits zugibt.

**14.** Verfahren nach einem der Ansprüche 1 bis 13, dadurch gekennzeichnet, daß man bei dem folgenden Oxidationsverfahrensschritt die Salpetersäure entweder in einer einzigen Zugabe oder portionsweise oder kontinuierlich durch Eintropfen in das Reaktionsmilieu einbringt, das das p-Nitrosophenol enthält.

**15.** Verfahren nach einem der Ansprüche 1 bis 14, dadurch gekennzeichnet, daß man die Salpetersäure vom Beginn an zusetzt, vorzugsweise zusammen mit der Zugabe des Phenols.

**16.** Verfahren nach einem der Ansprüche 1 bis 15, dadurch gekennzeichnet, daß man die Salpetersäure in situ ausgehend von Stickstoffperoxid (Distickstofftetroxid) herstellt, das auf eine Temperatur von 20 °C bis 40 °C erhitzt wird.

**17.** Verfahren zur Herstellung von N-Acetyl-p-aminophenol, dadurch gekennzeichnet, daß man in einem ersten Verfahrensschritt das Nitrophenol gemäß einem Verfahren nach den Ansprüchen 1 bis 16 herstellt und daß man in dem folgenden Verfahrensschritt das p-Nitrophenol reduziert, anschließend das erhaltene p-Aminophenol acetyliert.

## Claims

**1.** A process for the preparation of a p-nitrophenol by the nitrosation of a phenolic compound in the presence of sulphuric acid, followed by the oxidation of the p-nitrosophenol by nitric acid, characterised in that in the first nitrosation stage the concentration of sulphuric acid is at least equal to 60% and in the second stage, at the end of the oxidation reaction, the sulphuric acid concentration is at the most equal to 80%, which makes it possible to precipitate the p-nitrophenol which is then separated.

**2.** A process according to Claim 1, characterised in that the phenolic compound corresponds to general formula (I):

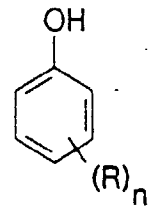

(I)

in which formula (I):

- R represents a hydrogen atom, an alkyl or alkoxy radical having 1 to 4 carbon atoms, preferably methyl or ethyl; a perfluorinated alkyl radical having 1 to 4 carbon atoms; a halogen atom, preferably chlorine, bromine or fluorine,
- n is a number equal to 0.1 or 2.

**3.** A process according to either claim 1 or claim 2, characterised in that the phenolic compound is phenol.

**4.** A process according to one of claims 1 to 3, characterised in that the sulphuric acid concentration in the nitrosation stage is between 60% and 90%, preferably between 70% and 80%.

**5.** A process according to one of claims 1 to 4, characterised in that the nitrosation agent is any $NO^+$ source, preferably nitrogen oxide NO associated with an oxidising agent, nitrogen dioxide $NO_2$, nitrogen trioxide $N_2O_3$, nitrogen peroxide $N_2O_4$, nitrous acid, nitrosyl sulphate or a nitrous salt, preferably an alkali metal salt and preferably sodium.

**6.** A process according to one of claims 1 to 5, characterised in that the quantity of nitrosation agent used is at least equal to the stoichiometric quantity, but is preferably in an excess up to 500% of the stoichiometric quantity, and preferably between 150% and 300%.

**7.** A process according to one of claims 1 to 6, characterised in that the concentration of phenolic substrate in the reaction medium is between 2% and 20% by weight.

**8.** A process according to one of claims 1 to 7, characterised in that the concentration of sulphuric acid in the oxidation stage is between 50% and 80%, preferably between 65% and 75%.

**9.** A process according to one of claims 1 to 8, characterised in that use is made of an aqueous solution of nitric acid or of a nitric acid precursor, preferably nitrogen peroxide.

**10.** A process according to one of claims 1 to 9, characterised in that the concentration of the aqueous solution of nitric acid is between 30% and 100%, preferably between 60% and 100% by weight.

**11.** A process according to one of claims 1 to 10, characterised in that the quantity of nitric acid, expressed by the molar ratio of phenol to nitric acid, varies between 0.9 and 1.2, preferably between 0.95 and 1.05.

**12.** A process according to one of claims 1 to 11, characterised in that the temperature is between 0 and 40°C, preferably between 10°C and 30°C.

**13.** A process according to any one of claims 1 to 12, characterised in that in the phenol nitrosation stage charging firstly takes place of the sulphuric acid solution, followed by the parallel addition of the phenol and the nitrosation agent; or introduction takes place of the sulphuric acid solution and the nitrosation agent followed by the addition of phenol; or parallel introduction takes place to a sediment of the phenol, on the one hand, and of the sulphuric acid and the nitrosation agent, on the other hand.

**14.** A process according to one of claims 1 to 13, characterised in that in the following oxidation stage the nitric acid is introduced all at once or gradually in portions or in continuous manner, by pouring, into the reaction medium comprising the p-nitrosophenol.

**15.** A process according to one of claims 1 to 14, characterised in that nitric acid is added from the outset, preferably in parallel with the addition of phenol.

**16.** A process according to one of claims 1 to 15, characterised in that the nitric acid is formed in situ from the nitrogen peroxide which is heated to a temperature of between 20°C and 40°C.

**17.** A process for the preparation of N-acetyl p-aminophenol, characterised in that in a first stage the p-nitrophenol is prepared in accordance with the process described in one of claims 1 to 16, and, in a following stage, reduction of the p-nitrophenol takes place, followed by acetylation of the p-aminophenol obtained.